# EUROPÄISCHE PATENTSCHRIFT

(11) **EP 1 899 510 B1**
(45) Veröffentlichungstag und Bekanntmachung des Hinweises auf die Patenterteilung: **12.11.2008**
(21) Anmeldenummer: 06762322.3
(22) Anmeldetag: 30.06.2006
(51) Int. Cl.: D01D 11/00, G01N 29/00

(54) **VORRICHTUNG ZUM HERSTELLEN EINES SPINNVLIESES**
DEVICE FOR PRODUCING A SPUN-BONDED NON-WOVEN
DISPOSITIF DE FABRICATION D'UN FILE-LIE

(30) Priorität: 05.07.2005 DE 102005031324
(43) Veröffentlichungstag der Anmeldung: 19.03.2008
(73) Patentinhaber: Oerlikon Textile GmbH & Co. KG, 42897 Remscheid (DE)
(72) Erfinder: SCHEMKEN, Matthias, 24536 Neumünster (DE); RAVE, Henning, 24239 Achterwehr (DE); SCHÖTTLER, Holger, 23795 Klein Rönnau (DE); MOOSHAMMER, Anton, 95213 Münchberg (DE)
(74) Vertreter: Kahlhöfer, Hermann
(86) Internationale Anmeldenummer: PCT/EP2006/006391
(87) Internationale Veröffentlichungsnummer: WO 2007/003377

(56) Entgegenhaltungen:
- DE-A1- 19 944 709
- US-A- 3 142 831
- PATENT ABSTRACTS OF JAPAN Bd. 005, Nr. 156 (C-074), 6. Oktober 1981 (1981-10-06) & JP 56 085409 A (UNITIKA LTD), 11. Juli 1981 (1981-07-11)

## Beschreibung

Die Erfindung betrifft eine Vorrichtung zum Schmelzspinnen und Abziehen einer Vielzahl von Filamenten gemäß dem Oberbegriff des Anspruchs 1 sowie ein Verfahren zum Schmelzspinnen und Abziehen einer Vielzahl von Filamenten gemäß dem Oberbegriff des unabhängigen Unteranspruchs.

Aus der WO 97/35053 ist eine gattungsgemäße Vorrichtung, in diesem Fall zum Herstellen eines Spinnvlieses, bekannt. Hierbei wird ein von einem Extruder zugeführtes schmelzflüssiges Polymer in einem Spinnbalken durch ein- oder mehrreihig linienförmig angeordnete Düsenbohrungen zu einer Vielzahl von Filamenten gesponnen. Auf die Filamente wird mittels einer in einem gewissen Abstand darunter angeordneten schlitzförmigen Abzugsdüse eine Zugkraft ausgeübt, die eine Verstreckung und Förderung der Filamente bewirkt. Dazu strömt aus der Innenwand der Abzugsdüse Druckluft in Förderrichtung der Filamente, wodurch die gewünschte Zugkraft auf die Filamente ausgeübt wird. Die Filamente legen sich auf einem unterhalb der Abzugdüse angeordneten Förderband in Wirrlage ab und bilden dort das Vlies.

Hohe Ansprüche an die Qualität des Endproduktes sowie an die Gleichmäßigkeit des Produktes erfordern bei automatisierter Produktion Maßnahmen zur Prozesskontrolle und -regelung. So zeigt die Patentanmeldung JP 07-216708 A einen Qualitätsregelkreis, bei in dem das produzierte Vlies mittels eines Sensors erfasst wird. Abhängig von dem Signal dieses Sensors bzw. der Abweichung des Signals von einem Referenzsignal werden Einstellungen an der Abzugsdüse vorgenommen. Als Sensor kommt hier eine CCD-Kamera zum Einsatz, die die Dichte des Vlieses misst. Dieses Messverfahren hat den Nachteil, dass das Qualitätsmerkmal des Produktes erst mit einer Verzögerung erfasst wird. Zudem ist die Sensorik sehr aufwändig und anfällig für Verschmutzungen.

Es ist daher Aufgabe der Erfindung, eine einfache und zuverlässige Sensorik zur Erfassung eines Qualitätsmerkmals bereit zu stellen, die das Messergebnis Verzögerungsfrei erfasst. Diese Aufgabe wird von einer Vorrichtung nach dem Anspruch 1 gelöst. Dazu wird die Abzugsdüse direkt mit einem Überwachungsmittel verbunden. Der Vorteil dieser Anordnung ist, dass das Überwachungsmittel direkt im Prozess misst. Somit stehen bei eventuellen Änderungen des Prozesses die Messergebnisse ohne Zeitverzögerung sofort zur Verfügung.

In einer bevorzugten Ausführungsform besteht das Überwachungsmittel aus einem oder mehreren Körperschallsensoren. Unter Körperschall versteht man transiente Schallwellen, die sich im Bauteil ausbreiten. Angeregt werden die Schallwellen durch die aerodynamischen Vorgänge innerhalb der Abzugsdüse. Eventuell auftretende Druckluftschwankungen oder Änderungen in der Beschaffenheit der Filamente haben einen starken Einfluss auf die aerodynamischen Vorgänge innerhalb der Abzugsdüse und können so leicht detektiert werden. Ebenso beeinflussen die durch die Abzugsdüse geführten Filamente die aerodynamischen Rahmenbedingungen. Somit können Veränderungen der Filamente indirekt erfasst werden. Der Vorteil der Erfindung liegt darin, dass der Körperschallsensor einfach installiert werden kann, ohne dass er direkt innerhalb des Strömungskanals der Abzugsdüse vorgesehen sein muss. Vielmehr ist es ausreichend, wenn der Sensor gut Körperschall leitend mit der Abzugsdüse verbunden ist. Für die Körperschallmessung geeignet ist ein Schwingungssensor, der insbesondere im hochfrequenten Bereich die mechanischen Schwingungen in elektrische Signale umwandelt.

Welche Anzahl von Körperschallsensoren tatsächlich eingesetzt wird, hängt von den baulichen Gegebenheiten ab. In Hinblick auf eine einfache Konstruktion ist ein einzelner Körperschallsensor zu bevorzugen. Da aber gattungsgemäße Abzugsdüsen in der Regel eine räumliche Ausdehnung aufweisen, die nicht durch die Empfindlichkeit eines einzelnen Sensors erfassbar ist, kann es erforderlich sein, auf der Abzugsdüse mehrere parallel geschaltete Körperschallsensoren vorzusehen.

Es ist zwar aus der WO 88/08047 bekannt, die Dichte eines durch eine Verengung geführten Filamentbündels mittels eines Mikrofons zu messen. Dabei wird das zu vermessende Filamentbündel durch eine trichterförmige Verengung geführt. Die dadurch zwischen den Filamenten untereinander sowie zwischen den Filamenten und der Wandung der Verengung hervorgerufene Reibung bewirkt in Verbindung mit der Bewegung des Filamentbündels eine Luftschallemission, die proportional zur Dichte des Filamentbündels ist. Hierdurch wird jedoch nicht die hier beschriebene Lösung nahe gelegt, da bei der hier der zu Grunde liegenden Aufgabenstellung eine Verzugsdüse mit Drucklufteinsatz verwendet wird. Bekanntermaßen bewirkt Druckluft störende Luftschallemissionen. Dieser Störpegel ist höher als das Nutzsignal, welches durch geringfügige Veränderungen der Prozessparameter hervorgerufen wird, was eine Schallmessung für einen Fachmann ausschließt. Zudem liegt bei der der Aufgabenstellung zugrunde liegenden Vorrichtung keine intensive Reibung zwischen den Filamenten untereinander oder zwischen den Filamenten und der Wandung vor.

Es hat sich gezeigt, dass ein besonders vorteilhafter Einbauort für die Körperschallsensoren der in Förderrichtung hintere Bereich der Abzugsdüse ist. Daher ist in einer bevorzugten Ausführungsform das Überwachungsmittel hier angeordnet. Dies hat zudem den Vorteil, dass die Körperschallsensoren leicht von der Unterseite der Abzugsdüse montiert werden können.

Das Messsignal des Überwachungsmittels wird in einer besonders vorteilhaften Weiterbildung der Erfindung dazu genutzt, mittels eines Steuergerätes den Istwert mit einem Sollwert zu vergleichen und bei Abweichungen ein Fehlersignal zu erzeugen. Dieses Fehlersignal kann beispielsweise ein Warnsignal für den Bediener sein. Ebenfalls kann erfindungsgemäß unter dem Fehlersignal eine Fehlermeldung an eine übergeordnete Steuerung verstanden werden. Unter Messsignal ist hier nicht nur das originäre Signal des Sensors zu verstehen. Vielmehr, insbesondere bei Einsatz eines Körperschallsensors, wird hier eine umfangreiche Signalvorverarbeitung vorausgesetzt um beispielsweise die in einem bestimmten Frequenzspektrum auftretende Signalleistung zu ermitteln. Andere zweckdienliche Formen der Signalvorverarbeitung sind dem Fachmann bekannt und werden von dieser Erfindung mit abgedeckt. Das Ergebnis dieser Vorverarbeitung des Signals wird dann mit dem Sollwert verglichen.

In einer Variante der Weiterbildung der Erfindung wird das Messsignal des Überwachungsmittels mittels eines Steuergerätes mit einem Sollwert verglichen und die Abweichung an eine übergeordnete Steuerung weitergeleitet, das Mittel aufweist, um daraufhin regelnd in den Prozess einzugreifen.

Eine bevorzugte Ausführungsform der erfindungsgemäßen Vorrichtung ist für die Herstellung von Spinnvlies vorgesehen. Aus diesem Grund weist der der Spinnbalken und die Abzugsdüse eine Ausstreckung quer zur Förderrichtung der Filamente in etwa über die Breite des Spinnvlieses auf. Dabei ist die Ausrichtung im Regelfall orthogonal zur Förderrichtung des Spinnvlieses. Der Spinnbalken oder die Abzugsdüse können aber auch unter einem Winkel bis herunter zu 45° zur Förderrichtung des Spinnvlieses angeordnet sein. In diesem Fall ergibt sich die Breite des Spinnvlieses aus der Projektion der wirksamen Breite des Spinnbalkens oder der Abzugsdüse in Förderrichtung des Spinnvlieses.

Ein erfindungsgemäßes Verfahren zum Schmelzspinnen und Abziehen von Filamenten sieht die Verfahrensschritte Zuführen von schmelzflüssigem Polymer, Extrudieren von Filamenten, Abziehen der Filamente in einem Luftstrom mit einer Abzugsdüse und das Messen eines Signals, das von einem an der Abzugsdüse angeordneten Überwachungsmittel gemessen wird, vor.

In einer bevorzugten Variante ist dies das Signal eines oder mehrerer an der Abzugsdüse vorgesehenen Körperschallsensoren.

Um dieses Signal weiter verarbeiten zu können, wird aus dem Signal zunächst eine charakteristische Kenngröße gebildet. Dies kann in einer bevorzugten Variante die Signalleistung sein. Die Signalleistung repräsentiert sehr gut die Intensität der Vorgänge im Prozeß, die für die Entstehung des Messsignals verantwortlich sind. Es sind dem Fachmann aber auch andere Kenngrößen bekannt wie Mittelwert, Effektivwert, Maximalwerte etc, die die Vorgänge ebenfalls repräsentieren. Diese äquivalenten Größen sind ebenfalls von der Erfindung abgedeckt.

Bei einer besonders bevorzugten Variante des Verfahrens wird die charakteristische Kenngröße innerhalb eines oder mehrerer Frequenzbereiche gebildet. So ist es sinnvoll, nur Frequenzbereiche oberhalb der Frequenz, die durch rotierende oder bewegte Maschinenteile angeregt werden, zu berücksichtigen. Ebenfalls ist es sinnvoll, diejenigen Frequenzbereiche auszublenden, die beispielsweise durch für die Überwachung uninteressante Luftströmungen angeregt werden. Welche Frequenzbereiche letztendlich für die Überwachung relevant sind, kann leicht experimentell bestimmt werden.

In einer alternativen Verfahrensvariante wird die charakteristische Kenngröße durch eine Frequenzanalyse gebildet werden. Hierzu zählt beispielsweise der Vergleich bestimmter Frequenzbereiche miteinander. Somit wird der Kennwert durch die Signalstärke in einem Frequenzbereich im Bezug zu der Signalstärke in einem zweiten, gegebenenfalls größeren Frequenzbereich gebildet. Dem Fachmann für Signalverarbeitung sind entsprechende Methoden der Frequenzanalyse bekannt. Welche Methode letztendlich zum Einsatz kommt, ist auch hier experimentell zu bestimmen, indem die Veränderungen im Frequenzbereich bei einer Störung gezielt analysiert wird.

Die durch die zuvor genannten Verfahrensalternativen gebildete Kenngröße wird daraufhin mit einem Referenzwert verglichen und bei Abweichung eine entsprechende Aktion ausgelöst.

Der Vergleich kann beispielsweise mit einem zuvor bestimmten und vorgegebenen Referenzwert erfolgen. Der Referenzwert kann auch im Prozess selbst ermittelt werden. So erfolgt der Vergleich der aktuell gemessenen Kenngröße mit den innerhalb eines bestimmten Zeitraumes gemessenen und gemittelte, gegebenenfalls gewichtet gemittelten, Kenngrößen.

Im Fall einer räumlich ausgestreckten Abzugsdüse wird in einer Verfahrensvariante bei mehreren räumlich getrennten Sensoren diese gruppiert und die Signale der Sensor-Gruppen untereinander verglichen, um so abnormale Zustände zu erkennen. Dabei kann eine Sensorgruppe einen oder mehrere Sensoren enthalten.

Die auszuführende Aktion ist eine Meldung des Ereignisses an den Bediener oder an eine übergeordnete Steuerung.

In einer bevorzugten Verfahrensvariante ist die auszuführende Aktion die Veränderung eines Verfahrensparameters des Spinnprozesses, über den die Vorgänge in der Abzugsdüse direkt oder indirekt beeinflusst werden. Durch entsprechende Veränderungen der Betriebsparameter des Extruders oder des beheizten Spinnbalkens mit integrierter Dosierpumpe können so Fördermenge, Förderdruck und Temperatur es Polymers im Sinne einer Regelung verändert werden. Diese Parameter beeinflussen die Vorgänge in der Abzugsdüse indirekt. Eine direkte Beeinflussung ergibt sich durch die Veränderung der der Abzugsdüse zugeführten Druckluft sowie der Geometrie der Abzugsdüse. Im Fall einer beispielsweise in der Breite veränderbaren Abzugsdüse kann so der Strömungsquerschnitt verändert werden.

Welche Verfahrensparameter letztendlich sinnvoll beeinflusst werden und welche charakteristische Kenngröße aus dem Messsignal des Überwachungsmittels gebildet wird, ist im Einzelfall durch einen Fachmann experimentell zu ermitteln. So ist es durchaus sinnvoll, auch verschiedene charakteristische Kenngrößen zu ermitteln, die jeweils unterschiedliche Aktionen hervorrufen.

Ein Ausführungsbeispiel wird im Folgenden unter Hinweis auf die beigefügten Zeichnungen näher beschrieben.

Es stellen dar:

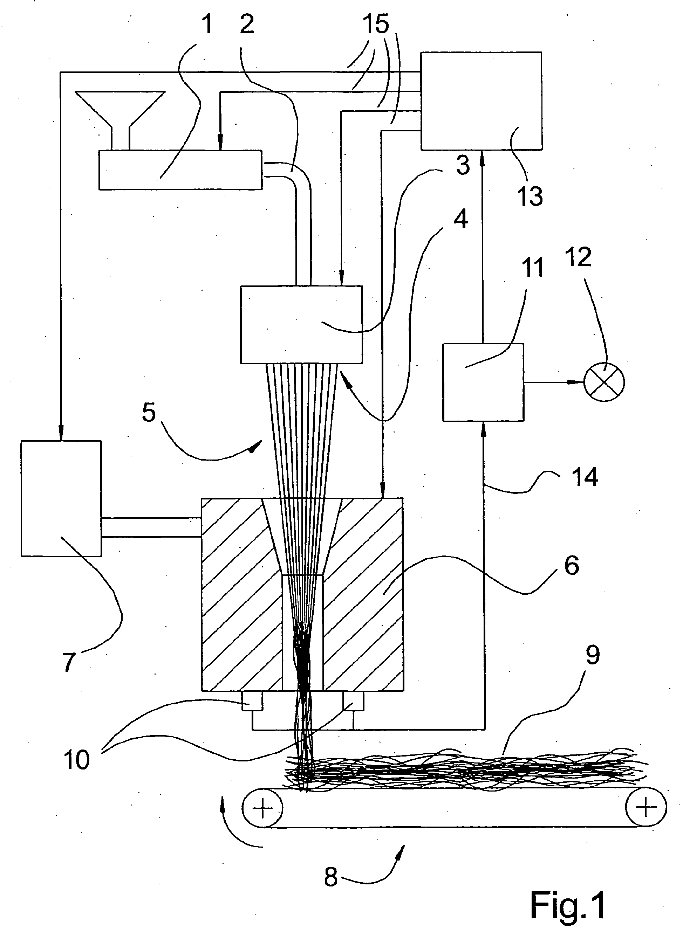

Fig. 1 Eine erfindungsgemäße Vorrichtung zum Herstellen eines Spinnvlieses

In Figur 1 ist eine erfindungsgemäße Vorrichtung zum Schmelzspinnen dargestellt. In diesem Beispiel handelt es sich um eine Vorrichtung zum Herstellen eines Spinnvlieses. Von einer Schmelzquelle 1, beispielsweise einem Extruder, wird schmelzflüssiges Polymer über eine Schmelzeleitung 2 dem Spinnbalken 3 zugeführt. Der Spinnbalken 3 erstreckt sich senkrecht zur Zeichenebene über eine Breite, die in etwa der Breite des herzustellenden Spinnvlieses entspricht. An seiner Unterseite weist der Spinnbalken 3 eine Vielzahl von Spinndüsenbohrungen 4 auf, durch die das schmelzflüssige Polymer unter hohem Druck gefördert und zu Filamenten 5 geformt wird. Der interne Aufbau des Spinnbalkens 3 weist Verteilerleitungen, Spinnpumpen zur Druckerhöhung sowie eine Beheizung auf und ist aus dem Stand der Technik bekannt.

Die aus dem Spinnbalken 3 austretenden Filamente 5 sind senkrecht zur Zeichenebene in Form eines Vorhanges angeordnet und werden einer Abzugsdüse 6 zugeführt. Die Abzugsdüse 6 erstreckt sich ebenfalls senkrecht zur Zeichenebene über eine Breite, die in etwa der Breite des herzustellenden Spinnvlieses entspricht. Von einer Druckluftquelle 7 wird der Abzugsdüse 6 Druckluft zugeführt, die mit hoher Geschwindigkeit durch die Düse in Förderrichtung strömt. Dadurch wird auf die Filamente 5 eine Zugkraft ausgeübt. Unterhalb der Abzugsdüse ist ein Förderband 8 angeordnet, auf dass die Filamente 5 von der Abzugsdüse 6 geworfen werden. Dabei wird das Spinnvlieses 9 gebildet, das durch das Förderband 8 kontinuierlich abtransportiert wird.

An der Unterseite der Abzugsdüse 6 ist ein Überwachungsmittel 10, hier in Form von mehreren Körperschallsensoren, vorgesehen. Der gegebenenfalls einer Signalvorverarbeitung unterzogene Messwert des Überwachungsmittels 10 wird einem Steuergerät 11 zugeführt. Die Signalvorverarbeitung kann das Bilden einer Kenngröße wie beispielsweise der Signalleistung in bestimmten Frequenzbereichen oder eine Frequenzanalyse sein. Das Steuergerät 11 vergleicht den Messwert mit einem Sollwert und führt im Fall einer Abweichung diese einem Signalmittel 12 zu. Dabei kann das Signalmittel 12 entweder eine Anzeige für den Bediener sein oder eine Verbindung zu einer übergeordneten Steuereinrichtung darstellen, der der Fehler gemeldet wird.

Alternativ führt das Steuergerät 11 bei einer Abweichung der Anlagensteuerung 13 ein die Größe der Abweichung repräsentierendes Stellsignal 15 zu. Die Anlagensteuerung 13 hat die Möglichkeit, abhängig von dem Stellsignal auf die Betriebsparameter verschiedener Bestandteile der Vorrichtung zum Herstellen eines Spinnvlieses einzuwirken. Dies sind beispielsweise Temperatur oder Drehzahl des Extruders (Schmelzquelle 1), Temperatur oder Pumpendruck des Spinnbalkens 3 sowie Druck oder Fördermenge der Druckluftquelle 7 etc. Ebenfalls möglich und vorgesehen ist eine direkte Beeinflussung der Abzugsdüse 6, indem zum Beispiel der Düsenquerschnitt mit geeigneten Aktoren verändert wird.

### Bezugszeichenliste

- 1: Schmelzequelle
- 2: Schmelzeleitung
- 3: Spinnbalken
- 4: Spinndüsenbohrungen
- 5: Filamente
- 6: Abzugsdüse
- 7: Druckluftquelle
- 8: Förderband
- 9: Vlies
- 10: Überwachungsmittel
- 11: Steuergerät
- 12: Signalmittel
- 13: Anlagensteuerung
- 14: Signal
- 15: Stellsignal

## Patentansprüche

1. Vorrichtung zum Schmelzspinnen und Abziehen einer Vielzahl von Filamenten (5), mit einer Schmelzequelle (1) zum Zuführen eines schmelzflüssigen Polymers, mit einem Spinnbalken (3) mit einer Vielzahl von Spinndüsenbohrungen (4) zum Extrudieren des schmelzflüssigen Polymers zu Filamenten (5), mit einer unterhalb der Spinnendüsenbohrungen (4) angeordneten Abzugsdüse (6), wobei die Filamente (5) durch die Abzugsdüse (6) führbar sind, und wobei die Abzugsdüse (6) derart ausgebildet ist, dass zum Abziehen auf die Filamente (5) mittels Druckluft eine Zugkraft ausübbar ist, **dadurch gekennzeichnet, dass** die Abzugsdüse (6) mit einem Überwachungsmittel (10) zur Überwachung des Abzugsvorganges verbunden ist.

2. Vorrichtung nach Anspruch 1, **dadurch gekennzeichnet, dass** das Überwachungsmittel (10) ein oder mehrere Körperschallsensoren ist.

3. Vorrichtung nach Anspruch 2, **dadurch gekennzeichnet, dass** die Körperschallsensoren (10) an dem in Förderrichtung der Filamente (5) gesehen hinteren Bereich der Abzugsdüse (6) vorgesehen sind und Schall leitend mit der Abzugsdüse (6) verbunden sind.

4. Vorrichtung nach einem der vorhergehenden Ansprüche, **dadurch gekennzeichnet, dass** das Überwachungsmittel (10) mit einem Steuergerät (11) verbunden ist, welches mit einem Signalmittel (12) zur Signalisierung von Abweichungen mit einem Referenzwert verbunden ist.

5. Vorrichtung nach einem der vorhergehenden Ansprüche, **dadurch gekennzeichnet, dass** das Überwachungsmittel (10) mit einem Steuergerät (11) verbunden ist, welches bei Abweichungen des vom Überwachungsmittel gemessenen Wertes von einem Sollwert ein Stellsignal (15) erzeugt, mittels dessen ein Parameter des Herstellungsprozesses verändert wird.

6. Vorrichtung nach einem der vorhergehenden Ansprüche, **dadurch gekennzeichnet, dass** zumindest eines der Vorrichtungsteile Spinnbalken (3) oder Abzugsdüse (6) eine Ausstreckung quer zur Förderrichtung der Filamente (5) aufweist.

7. Verfahren zum Abziehen einer Vielzahl von Filamenten (5), bei welchem die Filamente aus einem schmelzflüssigen Polymer extrudiert werden und bei welchem die Filamente (5) mittels einer durch eine Abzugsdüse (6) erzeugten Druckluftströmung abgezogen werden, **dadurch gekennzeichnet, dass** zumindest ein mit einem Überwachungsmittel (10) an der Abzugsdüse (6) gemessenes Messsignal zur Überwachung des Abzugsvorganges erfasst wird.

8. Verfahren nach Anspruch 7, **dadurch gekennzeichnet, dass** das Messsignal das Signal von einem oder mehreren Körperschallsensoren ist.

9. Verfahren nach Anspruch 7 oder 8, **dadurch gekennzeichnet, dass** aus dem Signal (14) des Überwachungsmittels (10) eine charakteristische Kenngröße gebildet wird.

10. Verfahren nach Anspruch 9, **dadurch gekennzeichnet, dass** die charakteristische Kenngröße die Signalleistung oder eine äquivalente Größe ist.

11. Verfahren nach Anspruch 9 oder 10, **dadurch gekennzeichnet, dass** die charakteristische Kenngröße innerhalb eines oder mehrerer Frequenzbänder gebildet wird.

12. Verfahren nach Anspruch 9, **dadurch gekennzeichnet, dass** die charakteristische Kenngröße durch eine Frequenzanalyse gebildet wird.

13. Verfahren nach einem der Ansprüche 9 bis 12, **dadurch gekennzeichnet, dass** die charakteristische Kenngröße mit einem Referenzwert verglichen wird und bei Abweichungen vom Referenzwert eine Aktion ausgelöst wird.

14. Verfahren nach Anspruch 13, **dadurch gekennzeichnet, dass** der Referenzwert ein vorgegebener Grenzwert ist.

15. Verfahren nach Anspruch 13, **dadurch gekennzeichnet, dass** der Referenzwert laufend aus zuvor ermittelten charakteristischen Kenngröße gebildet wird.

16. Verfahren nach einem der Ansprüche 9 bis 13, **dadurch gekennzeichnet, dass** das Überwachungsmittel aus mehreren Sensoren gebildet wird, die jeweils mehrere Gruppen bilden, und dass der Referenzwert einer Gruppe die charakteristische Kenngröße einer anderen Gruppe ist.

17. Verfahren nach einem der Ansprüche 13 bis 16, **dadurch gekennzeichnet, dass** die Aktion die Meldung des Ereignisses ist.

18. Verfahren nach einem der Ansprüche 13 bis 16, **dadurch gekennzeichnet, dass** die Aktion die Veränderung eines Verfahrensparameters ist.

19. Verfahren nach Anspruch 18, **dadurch gekennzeichnet, dass** der Verfahrensparameters die Fördermenge oder der Förderdruck des Polymers ist.

20. Verfahren nach Anspruch 18, **dadurch gekennzeichnet, dass** der Verfahrensparameter die Temperatur des Polymers ist.

21. Verfahren nach Anspruch 18, **dadurch gekennzeichnet, dass** der Verfahrensparameter der Druck oder die Fördermenge der der Abzugsdüse zugeführten Druckluft ist.

22. Verfahren nach Anspruch 18, **dadurch gekennzeichnet, dass** der Verfahrensparameter eine geometrische Kenngröße, insbesondere die Querschnittsfläche der Abzugsdüse ist.

23. Verfahren nach einem der Ansprüche 9 bis 22, **dadurch gekennzeichnet, dass** mehrere charakteristische Kenngrößen gebildet werden.

24. Verfahren nach einem der Ansprüche 13 bis 23, **dadurch gekennzeichnet, dass** mehrere Aktionen ausgelöst werden.

## Claims

1. A device for the melt-spinning and draw-off of a multiplicity of filaments (5), with a melt source (1) for supplying a molten polymer, with a spinning beam (3) having a multiplicity of spinneret bores (4) for extruding the molten polymer into filaments (5), and with a draw-off nozzle (6) arranged beneath the spinneret bores (4), the filaments (5) being guidable through the draw-off nozzle (6), and the draw-off nozzle (6) being designed in such a way that, for the draw-off, a tensile force is exertable on the filaments (5) by means of compressed air, **characterized in that** the draw-off nozzle (6) is connected to a monitoring means (10) for monitoring the draw-off operation.

2. The device as claimed in claim 1, **characterized in that** the monitoring means (10) is one or more structure-borne sound sensors.

3. The device as claimed in claim 2, **characterized in that** the structure-borne sound sensors (10) are provided on that region of the draw-off nozzle (6) which is at the rear, as seen in the conveying direction of the filaments (5), and are connected to the draw-off nozzle (6) so as to conduct sound.

4. The device as claimed in one of the preceding claims, **characterized in that** the monitoring means (10) is connected to a control apparatus (11) which is connected to a signal means (12) for signaling deviations from a reference value.

5. The device as claimed in one of the preceding claims, **characterized in that** the monitoring means (10) is connected to a control apparatus (11) which, in the event of deviations of the value measured by the monitoring means from a desired value, generates a collecting signal (15), by means of which a parameter of the production process is varied.

6. The device as claimed in one of the preceding claims, **characterized in that** at least one of the device parts, spinning beam (3) or draw-off nozzle (6), has an extent transverse to the conveying direction of the filaments (5).

7. A method for drawing-off a multiplicity of filaments (5), in which the filaments are extruded from a molten polymer, and in which the filaments (5) are drawn off by means of a compressed air flow generated by a draw-off nozzle (6), **characterized in that** at least one measurement signal measured by a monitoring means (10) on the draw-off nozzle (6) is detected for the purpose of monitoring the draw-off operation.

8. The method as claimed in claim 7, **characterized in that** the measurement signal is the signal from one or more structure-borne sound sensors.

9. The method as claimed in claim 7 or 8, **characterized in that** a characteristic quantity is formed from the signal (14) from the monitoring means (10).

10. The method as claimed in claim 9, **characterized in that** the characteristic quantity is the signal power or an equivalent quantity.

11. The method as claimed in claim 9 or 10, **characterized in that** the characteristic quantity is formed within one or more frequency bands.

12. The method as claimed in claim 9, **characterized in that** the characteristic quantity is formed by means of frequency analysis.

13. The method as claimed in one of claims 9 to 12, **characterized in that** the characteristic quantity is compared with a reference value and, in the event of deviations from the reference value, an action is triggered.

14. The method as claimed in claim 13, **characterized in that** the reference value is a stipulated limit value.

15. The method as claimed in claim 13, **characterized in that** the reference value is formed continuously from a predetermined characteristic quantity.

16. The method as claimed in one of claims 9 to 13, **characterized in that** the monitoring means is formed from a plurality of sensors which in each case form a plurality of groups, and **in that** the reference value of one group is the characteristic quantity of another group.

17. The method as claimed in one of claims 13 to 16, **characterized in that** the action is the communication of the event.

18. The method as claimed in one of claims 13 to 16, **characterized in that** the action is the variation of a method parameter.

19. The method as claimed in claim 18, **characterized in that** the method parameter is the delivery or the conveying pressure of the polymer.

20. The method as claimed in claim 18, **characterized in that** the method parameter is the temperature of the polymer.

21. The method as claimed in claim 18, **characterized in that** the method parameter is the pressure or the delivery of the compressed air supplied to the draw-off nozzle.

22. The method as claimed in claim 18, **characterized in that** the method parameter is a geometric characteristic quantity, in particular the cross-sectional area of the draw-off nozzle.

23. The method as claimed in one of claims 9 to 22, **characterized in that** a plurality of characteristic quantities are formed.

24. The method as claimed in one of claims 13 to 23, **characterized in that** a plurality of actions are triggered.

## Revendications

1. Dispositif pour le filage à l'état fondu et pour le défilage d'une multiplicité de filaments (5), avec une source de fonte (1) pour alimenter un polymère liquide en fusion, avec une barre de filage (3) ayant une multiplicité de trous de filières (4) pour extruder le polymère liquide en fusion en filaments (5), avec une buse de défilage (6) agencée en dessous des trous de filière (4), les filaments (5) pouvant être guidés à travers la buse de défilage (6) et la buse de défilage (6) étant réalisée de manière telle que pour le défilage une force de traction peut être exercée au moyen d'air comprimé sur les filaments (5), **caractérisé en ce que** la buse de défilage (6) est connectée à un moyen de contrôle (10) pour contrôler l'opération de défilage.

2. Dispositif selon la revendication 1, **caractérisé en ce que** le moyen de contrôle (10) est un ou plusieurs capteurs de bruits de structure.

3. Dispositif selon la revendication 2, **caractérisé en ce que** les capteurs de bruits de structure (10) sont prévus dans la région arrière de la buse de défilage (6), vu dans le sens d'alimentation des filaments (5), et sont reliés à la buse de défilage (6) de manière à transmettre le son.

4. Dispositif selon l'une des revendications précédentes, **caractérisé en ce que** le moyen de contrôle (10) est en connexion avec un appareil de commande (11) qui est connecté à un moyen de signal (12) pour signaliser des écarts avec une valeur de référence.

5. Dispositif selon l'une des revendications précédentes, **caractérisé en ce que** le moyen de contrôle (10) est relié à un appareil de commande (11) qui en cas d'écarts de la valeur mesurée par le moyen de contrôle d'une valeur de consigne, génère un signal collectif (15), au moyen duquel un paramètre du procédé de fabrication est modifié.

6. Dispositif selon l'une des revendications précédentes, **caractérisé en ce qu'**au moins une des parties de dispositifs, barre de filage (3) ou buse de défilage (6) a une extension transversale par rapport au sens d'alimentation des filaments (5).

7. Procédé pour le défilage d'une multiplicité de filaments (5), dans lequel les filaments sont extrudés d'un polymère liquide en fusion et dans lequel les filaments (5) sont défilés à l'aide d'un flux d'air comprimé généré par une buse de défilage (6), **caractérisé en ce que** pour contrôler l'opération de défilage au moins un signal de mesure mesuré avec un moyen de contrôle (10) sur la buse de défilage (6) est relevé.

8. Procédé selon la revendication 7, **caractérisé en ce que** le signal de mesure est le signal d'un capteur de bruits de structure ou d'une pluralité de ceux-ci.

9. Procédé selon la revendication 7 ou 8, **caractérisé en ce qu'**un paramètre caractéristique est formé à partir du signal (14) du moyen de contrôle (10).

10. Procédé selon la revendication 9, **caractérisé en ce que** le paramètre caractéristique est la puissance de signal ou un paramètre équivalent.

11. Procédé selon la revendication 9 ou 10, **caractérisé en ce que** le paramètre caractéristique est formé à l'intérieur d'une bande de fréquence ou plusieurs bandes de fréquence.

12. Procédé selon la revendication 9, **caractérisé en ce que** le paramètre caractéristique est formé par une analyse de fréquences.

13. Procédé selon l'une des revendications 9 à 12, **caractérisé en ce que** le paramètre caractéristique est comparé avec une valeur de référence et qu'en cas d'écarts de la valeur de référence une action est déclenchée.

14. Procédé selon la revendication 13, **caractérisé en ce que** la valeur de référence est une valeur limite prédéterminée.

15. Procédé selon la revendication 13, **caractérisé en ce que** la valeur de référence est formée continuellement à partir d'un paramètre caractéristique déterminé préalablement.

16. Procédé selon l'une des revendications 9 à 13, **caractérisé en ce que** le moyen de contrôle est formé de plusieurs capteurs, que forment respectivement plusieurs groupes et **en ce que** la valeur de référence d'un groupe est le paramètre caractéristique d'un autre groupe.

17. Procédé selon l'une des revendications 13 à 16, **caractérisé en ce que** l'action est l'avis de l'évènement.

18. Procédé selon l'une des revendications 13 à 16, **caractérisé en ce que** l'action est le changement d'un paramètre de procédé.

19. Procédé selon la revendication 18, **caractérisé en ce que** le paramètre de procédé est le débit de transport ou la pression de transport du polymère.

20. Procédé selon la revendication 18, **caractérisé en ce que** le paramètre de procédé est la température du polymère.

21. Procédé selon la revendication 18, **caractérisé en ce que** le paramètre de procédé est la pression ou le débit de transport de l'air comprimé alimenté dans la buse de défilage.

22. Procédé selon la revendication 18, **caractérisé en ce que** le paramètre de procédé est un paramètre caractéristique géométrique, notamment l'aire en section transversale de la buse de défilage.

23. Procédé selon l'une des revendications 9 à 22, **caractérisé en ce que** plusieurs paramètres caractéristiques sont formés.

24. Procédé selon l'une des revendications 13 à 23, **caractérisé en ce qu'**une pluralité d'actions est déclenchée.
